# EUROPEAN PATENT APPLICATION

(11) **EP 0 965 353 A1**
(43) Date of publication of application: **22.12.1999**
(21) Application number: 99201899.4
(22) Date of filing: 15.06.1999
(51) Int. Cl.: A61L 2/26

(54) **Container for storing sensitive waste-products**

(30) Priority: 17.06.1998 NL 1009426; 11.03.1999 NL 1011539
(71) Applicant: Van Egmond Technische Handelsonderneming B.V., 3861 RH Nijkerk (NL)
(72) Inventor: Versluis, Evert Cornelis Antonie, 3862 WL Nijkerk (NL)
(74) Representative: Aalbers, Arnt Reinier

(57) **Abstract**

A container (1) for storing sensitive waste products, in particular hospital waste, can be closed with a cover (2) and is provided with a bottom and an upright wall or upright walls, which defines or define an open upper side which can be closed with said cover (2). The container (1) and the cover (2) are made of a plastic material. The cover (2) and/or the container (1) is (are) provided with connecting means (3), which are capable of effecting a connection between the inside of the container (1) and the surrounding atmosphere when the container (1) is closed by the cover (2). The connecting means (3) comprise an element which is made of a plastic material which will shrink at an elevated temperature.

## Description

The invention relates to a container for storing sensitive waste products, in particular hospital waste, which container can be closed with a cover and which is provided with a bottom and an upright wall or upright walls, which defines or define an open upper side which can be closed with said cover, which container and which cover are made of a plastic, wherein said cover and/or said container is (are) provided with connecting means for effecting a connection between the inside of the container and the surrounding atmosphere when the container is closed by the cover.

Containers of this kind, which are known in various embodiments thereof, are for example disclosed in US-A-3,840,152, US-A-3,848,767 and EP-A-0 168 877. In particular when the known container is used for storing hospital waste therein, it is usual to transport the container, once it has been closed, to a waste processing facility. Furthermore, containers of this type are known wherein the container and its contents are sterilized at the location where it is used, for example at the hospital. To this end, the known container is provided with a cover having a small opening therein, which opening is closed by means of a special plug, which plug is pierced by an injection needle so as to inject water into the container for said sterilization. Then the contents and the water are sterilized by means of microwave radiation. This known container is made of a high-quality plastic, so that the container can be reused a number of times indeed, but which also makes the container costly to produce. Moreover, the sterilization process requires a relatively complicated special installation.

The object of the invention is to provide a container of the kind referred to in the introduction, which has been so designed that it is possible to disinfect or sterilized the contents of the container in a simple and effective manner.

In order to accomplish that objective, the container according to the invention is characterized in that said connecting means comprise an element which is made of a plastic material which will shrink at an elevated temperature.

In this manner a container is obtained wherein the high temperature during the disinfestation or sterilization process makes the waste products which are present inside the container accessible for said process without any additional operations being required.

Further it is noted that German Offenlegungsschrift No 28 10 457 discloses a cover plate for covering an opening in a wall, which must be painted, for example. A self-adhesive plate is used for this purpose, which will become detached from the treated object again as a result of shrinking under the influence of heat. DE OS 28 10 457 therefore relates to the protection of a surface to be treated. The airtight sealing of a container for storing sensitive waste products is not mentioned or suggested in said document. Further it is noted that European patent application (not pre-published) No. 0 891 785 discloses a "Schmelzplatte" (melting plate).

The invention will be explained in more detail hereafter with reference to drawings which schematically show a few embodiments of the container according to the invention.
Fig. 1 is a schematic, perspective view of a container according to the invention for storing sensitive waste products.
Fig. 2 is a schematic cross-sectional view of a cover of a container according to the invention, wherein a closing plug is used as the closing member.
Fig. 3 is a schematic plan view of a plug provided with an interrupted, projecting edge.
Fig. 4 is a schematic cross-sectional view of the plug of Fig. 3, which is anchored in a cover.

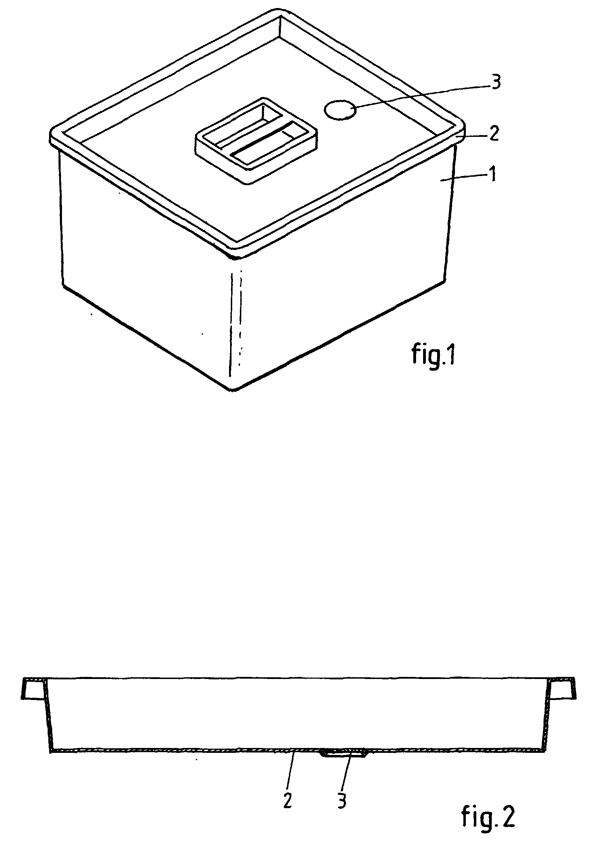

Figs. 1 and 2 show a container 1 for storing sensitive waste products, such as hospital waste, for example. Container 1 is of conventional design and comprises a bottom and upright walls, which define an open upper side to be closed by means of a cover 2. Cover 2 can be placed loosely on top of the open upper side of container 1 during use, whereby the container may or may not be temporarily closed. When container 1 is full, cover 2 is fixedly connected to the container, after which the container can be temporarily stored.

An opening of approximately 600 mm² is formed in cover 2, which opening is closed by a plug 3. Plug 3 is made of a plastic material which will shrink at an elevated temperature, a so-called heat shrinkable plastic, such as polyethylene, for example. As a result, said plug will automatically be removed from the opening during the sterilization process, for example in an autoclave, due to the temperature increase, and the shrunken plug 12 will fall into the container.

When the waste is to be processed, container 1 is sterilized by placing the container, usually together with a number of other containers, in an autoclave, whereby the contents are sterilized by means of steam sterilization at a temperature of ± 130 C. The steam can penetrate deep into the waste via the opening(s). Preferably, a vacuum is generated in the autoclave after plug 3 has shrunk as a result of being heated and the opening has been released, after which hot steam is injected into the autoclave space, so that the steam can penetrate deep into the waste. The cycle of generating a vacuum and then injecting steam can be repeated a few times, if desired, so that sterility of all the waste in the containers is ensured.

After completion of the sterilization process, the above-described container and its contents can be reduced in a shredder, for which purpose the container is for example configured in the manner disclosed in the present applicant's Dutch patent application No. 1007679.

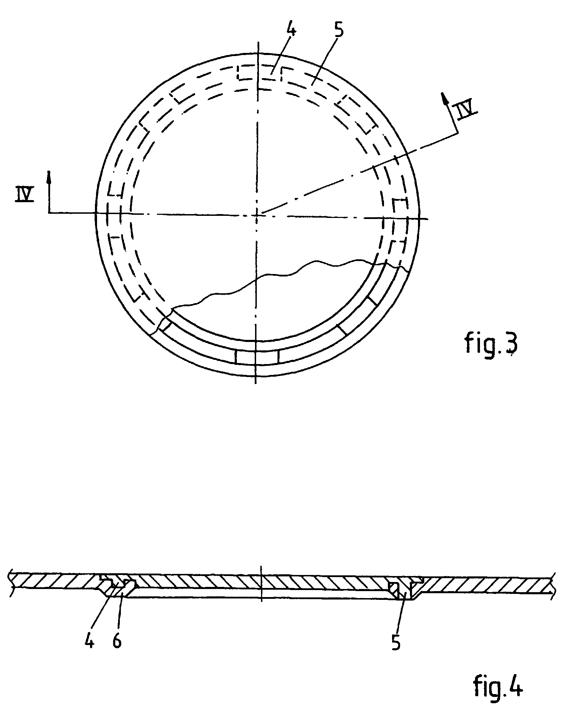

Figs. 3 and 4 show a preferred variant of cover 2. Also in this embodiment, plug 3 is made of a plastic material which will shrink at an elevated temperature, for example LDPE (Low Density PolyEthylene). Plug 3 is provided on the underside, near its circumference, with a projecting tongue or edge 4, which is anchored in a complementary grooved edge 6 in cover 2, which grooved edge 6 extends along the entire circumference of the opening which is closed by means of plug 3.

Edge 4 comprises eight equidistant sections 5, which project further than the rest of edge 4, such that the height of said sections is at least equal to the thickness of the grooved edge 6 that is formed in cover 2. In other words, the equidistant sections 5 extend through openings in grooved edge 6, as a result of which the anchoring becomes stronger. The anchoring may be additionally strengthened in that sections 5 surround the underside of edge 6 entirely or partially.

In the illustrated embodiment, edge 6 is recessed, which reduces the risk of damage being done by objects outside the container. The upper side of the plug may be positioned entirely or largely below the level of the upper side of the cover.

In order reduce the risk of damage being done by objects which are present in the container, or even prevent said damage altogether, a plate or cap (provided with at least one opening for passing air and steam therethrough) may be fitted on the inside of the cover to protect the underside of the plug.

The variant according to Figs. 3 and 4 is preferably formed by "dual injection moulding". This provides a very good, airtight seal which can easily be broken by raising the temperature to a sufficiently high level.

Instead of using a plug, it is also possible to form a portion of the cover of a heat shrinkable plastic, which portion will form an opening in the cover by shrinking.

It is also possible, of course, to provide one or more of the above-described features in the container itself.

The invention is not restricted to the above-described embodiments, which can be varied in several ways without departing from the scope of the invention. In this connection it is noted that the term autoclave space is understood to mean any space in which the above-described method can be carried out. Furthermore it is also possible to use a disinfecting process at a lower temperature instead of the above-described sterilization process.

## Claims

1. A container for storing sensitive waste products, in particular hospital waste, which container can be closed with a cover and which is provided with a bottom and an upright wall or upright walls, which defines or define an open upper side which can be closed with said cover, which container and which cover are made of a plastic, wherein said cover and/or said container is (are) provided with connecting means for effecting a connection between the inside of the container and the surrounding atmosphere when the container is closed by the cover, **characterized in that** said connecting means comprise an element which is made of a plastic material which will shrink at an elevated temperature.

2. A container according to claim 1, wherein said connecting means comprise at least one opening in said cover and/or in said container, which opening is closed by a closing member, which is made of a plastic material which will shrink at an elevated temperature.

3. A container according to claim 2, wherein said closing member is fitted at such a location in the container or in the cover that it will fall into the container upon shrinking.

4. A container according to claim 2 or 3, wherein said opening is closed by a plug which is made of a plastic material which will shrink at an elevated temperature.

5. A container according to claim 4, wherein said plug is provided on its underside with a projecting tongue or edge which is anchored in a complementary grooved edge surrounding said opening.

6. A container according to claim 5, wherein said projecting tongue or edge extends along the entire circumference of said plug.

7. A container according to claim 6, wherein said tongue or edge comprises a number of sections, which project further than the rest of said tongue or edge.

8. A container according to claim 7, wherein the height of said sections is at least equal to the thickness of the complementary grooved edge in the cover.

9. A container according to any one of the claims 4 - 8, wherein the upper side of said plug is positioned entirely or largely below the level of the upper side of the cover.
